# EUROPÄISCHE PATENTSCHRIFT

(11) **EP 2 945 921 B1**
(45) Veröffentlichungstag und Bekanntmachung des Hinweises auf die Patenterteilung: **22.03.2017**
(21) Anmeldenummer: 14700462.6
(22) Anmeldetag: 13.01.2014
(51) Int. Cl.: C07C 5/48, C07C 7/08, C07C 7/11, C07C 11/167

(54) **VERFAHREN ZUR HERSTELLUNG VON BUTADIEN DURCH OXIDATIVE DEHYDRIERUNG VON N-BUTENEN MIT ÜBERWACHUNG DES PEROXID-GEHALTS BEI DER PRODUKTAUFARBEITUNG**
METHOD FOR PRODUCING BUTADIENE BY OXIDATIVE DEHYDROGENATION OF N-BUTENES BY MONITORING THE PEROXIDE-CONTENT DURING PRODUCT PROCESSING
PROCÉDÉ DE PRÉPARATION DE BUTADIÈNE PAR DÉSHYDROGÉNATION OXYDATIVE DE N-BUTÈNES, AVEC SURVEILLANCE DE LA TENEUR EN PEROXYDE LORS DE L'ÉLABORATION DU PRODUIT

(30) Priorität: 16.01.2013 EP 13151491
(43) Veröffentlichungstag der Anmeldung: 25.11.2015
(73) Patentinhaber: BASF SE, 67056 Ludwigshafen am Rhein (DE)
(72) Erfinder: JOSCH, Jan Pablo, 67434 Neustadt (DE); GRÜNE, Philipp, 68161 Mannheim (DE); WALSDORFF, Christian, 67061 Ludwigshafen (DE)
(86) Internationale Anmeldenummer: PCT/EP2014/050451
(87) Internationale Veröffentlichungsnummer: WO 2014/111331

(56) Entgegenhaltungen:
- WO-A1-2014/111409
- JP-A- 2011 006 381
- Jacob Zabicky: "Analytical and Safety Aspects of Organic Peroxides and Related Functional Groups" In: "PATAI'S Chemistry of Functional Groups", 15. Dezember 2009 (2009-12-15), John Wiley & Sons, Ltd, Chichester, UK, XP055112656, ISBN: 978-0-47-068253-1 DOI: 10.1002/9780470682531.pat0353, Abschnitte 4.B.1, 4.B.2 und 4.E

## Beschreibung

Die Erfindung betrifft ein Verfahren zur Herstellung von Butadien durch oxidative Dehydrierung von n-Butenen, bei dem der Peroxid-Gehalt bei der Produktaufarbeitung überwacht wird.

Butadien ist eine bedeutende Grundchemikalie und wird beispielsweise zur Herstellung von Synthesekautschuken (Butadien-Homopolymere, Styrol-Butadien-Kautschuk oder Nitril-Kautschuk) oder zur Herstellung von thermoplastischen Terpolymeren (AcrylnitrilButadien-Styrol-Copolymere) eingesetzt. Butadien wird ferner zu Sulfolan, Chloropren und 1,4-Hexamethylendiamin (über 1,4-Dichlorbuten und Adipinsäuredinitril) umgesetzt. Durch Dimerisierung von Butadien kann ferner Vinylcyclohexen erzeugt werden, welches zu Styrol dehydriert werden kann.

Butadien kann durch thermische Spaltung (Steam-Cracken) gesättigter Kohlenwasserstoffe hergestellt werden, wobei üblicherweise von Naphtha als Rohstoff ausgegangen wird. Beim Steam-Cracken von Naphtha fällt ein Kohlenwasserstoff-Gemisch aus Methan, Ethan, Ethen, Acetylen, Propan, Propen, Propin, Allen, Butanen, Butenen, Butadien, Butinen, Methylallen, C₅- und höheren Kohlenwasserstoffen an.

Butadien kann auch durch oxidative Dehydrierung von n-Butenen (1-Buten und/oder 2-Buten) erhalten werden. Als Ausgangsgasgemisch für die oxidative Dehydrierung (Oxidehydrierung, ODH) von n-Butenen zu Butadien kann jedes beliebige n-Butene enthaltende Gemisch benutzt werden. Beispielsweise kann eine Fraktion verwendet werden, die als Hauptbestandteil n-Butene (1-Buten und/oder 2-Buten) enthält und aus der C₄-Fraktion eines Naphtha-Crackers durch Abtrennen von Butadien und Isobuten erhalten wurde. Des Weiteren können auch Gasgemische als Ausgangsgas eingesetzt werden, die 1-Buten, cis-2-Buten, trans-2-Buten oder deren Gemische umfassen und durch Dimerisierung von Ethylen erhalten wurden. Ferner können als Ausgangsgas n-Butene enthaltende Gasgemische eingesetzt werden, die durch katalytisches Wirbelschichtcracken (Fluid Catalytic Cracking, FCC) erhalten wurden.

Verfahren zur oxidativen Dehydrierung von Butenen zu Butadien sind grundsätzlich bekannt.

US 2012/0130137A1 beispielsweise beschreibt ein solches Verfahren unter Verwendung von Katalysatoren die Oxide von Molybdän, Bismuth und in der Regel weiteren Metallen enthalten. Für die nachhaltige Aktivität solcher Katalysatoren für die oxidative Dehydrierung ist ein kritisches Mindestmaß an Sauerstoffpartialdruck in der Gasatmosphäre erforderlich, um eine zu weitgehende Reduktion und damit Performanceverlust der Katalysatoren zu vermeiden. Aus diesem Grund kann in der Regel auch nicht mit einem stöchiometrischen Sauerstoffeinsatz oder vollständigem Sauerstoff-Umsatz im Oxidehydrierungsreaktor gearbeitet werden. In der US 2012/0130137 werden zum Beispiel ein Sauerstoffgehalt von 2,5 - 8 Vol.-% im Ausgangsgas beschrieben.

Die Notwendigkeit eines Sauerstoffüberschusses für solche Katalysatorsysteme ist allgemein bekannt und schlägt sich in den Test- oder Verfahrensbedingungen solcher Katalysatoren nieder. Stellvertretend seien die neueren Arbeiten von Jung et al. (Catal. Surv. Asia 2009, 13, 78-93; DOI 10.1007/s10563-009-9069-5 und Applied Catalysis A: General 2007, 317, 244-249; DOI 10.1016/j.apcata.2006.10.021) genannt.

Das Vorliegen von Sauerstoff neben Butadien nach der Reaktorstufe im Aufarbeitungsteil solcher Verfahren muss jedoch grundsätzlich als Risiko betrachtet werden. Insbesondere in flüssiger Phase bei ist die Bildung und Anreicherung organischer Peroxide zu prüfen. Diese Risiken wurden zum Beispiel von D. S. Alexander (Industrial and Engineering Chemistry 1959, 51, 733-738) diskutiert.

In der JP 2011-006381 A der Firma Mitsubishi wird das Risiko von Peroxidbildung im Aufarbeitungsteil eines Verfahrens zur Herstellung von konjugierten Alkadienen angesprochen. Zur Lösung wird der Zusatz von Polymerisationsinhibitoren zu den Absorptionslösungen und die Einstellung eines maximalen Peroxidgehalts von 100 Gewichtsppm durch Erhitzen der Absorptionslösungen beschrieben. Es werden auch Analysenwerte für Peroxid in den Beispielen zitiert. Allerdings fehlen jegliche Angaben zur Bestimmungsmethode solcher organischer Peroxide. Iodometrische Methoden, wie sie auch in handelsüblichen Prüfstäbchen für Peroxide Verwendung finden, sind regelmäßig nicht für die Bestimmung von komplexeren organischen Peroxiden, beispielsweise von oligomeren Peroxiden oder Verbindungen mit Dialkylperoxid-Gruppen geeignet. Auch kann die Genauigkeit solcher Methoden durch die Gegenwart anderer Bestandteile der Absorptionslösungen deutlich eingeschränkt sein. Auch Butadien selbst kann hier beispielsweise störend wirken.

Aufgabe der vorliegenden Erfindung ist es deshalb, ein Verfahren zur Bestimmung und Überwachung von Peroxidgehalten im Aufarbeitungsteil von Verfahren zur oxidativen Dehydrierung von n-Butenen zu Butadien zu finden. Vorzugsweise sollte dieses Verfahren auch automatisiert und weitgehend kontinuierlich oder halbkontinuierlich durchführbar sein.

Gelöst wird die Aufgabe durch ein Verfahren zur Herstellung von Butadien aus n-Butenen mit den folgenden Schritten:
A) Bereitstellung eines n-Butene enthaltenden Einsatzgasstroms a;
B) Einspeisung des n-Butene enthaltenden Einsatzgasstromes a und eines sauerstoffhaltigen Gases in mindestens eine Dehydrierzone und oxidative Dehydrierung von n-Butenen zu Butadien, wobei ein Produktgasstrom b enthaltend Butadien, nicht umgesetzte n-Butene, Wasserdampf, Sauerstoff, leicht siedende Kohlenwasserstoffe, gegebenenfalls Kohlenstoffoxide und gegebenenfalls Inertgase erhalten wird;
C) Abkühlung und Kompression des Produktgasstroms b in mindestens einer Abkühlstufe und mindestens einer Kompressionsstufe, wobei der Produktgasstrom b mit mindestens einem im Kreis geführtem organischen Lösungsmittel als Kühlmittel in Kontakt gebracht wird, wobei mindestens ein Kondensatstrom c1 enthaltend Wasser und ein Gasstrom c2 enthaltend Butadien, n-Butene, Wasserdampf, Sauerstoff, leicht siedende Kohlenwasserstoffe, gegebenenfalls Kohlenstoffoxide und gegebenenfalls Inertgase erhalten wird;
D) Abtrennung von nicht kondensierbaren und leicht siedenden Gasbestandteilen umfassend Sauerstoff, leicht siedende Kohlenwasserstoffe, gegebenenfalls Kohlenstoffoxide und gegebenenfalls Inertgase als Gasstrom d2 aus dem Gasstrom c2 durch Absorption der C₄-Kohlenwasserstoffe umfassend Butadien und n-Butene in mindestens einem im Kreis geführten Absorptionsmittel, wobei mindestens ein mit C₄-Kohlenwasserstoffen beladener Absorptionsmittelstrom und der Gasstrom d2 erhalten werden, und anschließende Desorption der C₄-Kohlenwasserstoffe aus dem beladenen Absorptionsmittelstrom, wobei ein C₄-Produktgasstrom d1 erhalten wird;
E) Auftrennung des C₄-Produktstroms d1 durch Extraktivdestillation mit einem für Butadien selektiven Lösungsmittel in einen Butadien und das selektive Lösungsmittel enthaltenden Stoffstrom e1 und einen n-Butene enthaltenden Stoffstrom e2;
F) Destillation des Butadien und das selektive Lösungsmittel enthaltenden Stoffstroms e1 in einen im Wesentlichen aus dem selektiven Lösungsmittel bestehenden Stoffstrom f1 und einen Butadien enthaltenden Stoffstrom f2;
wobei dem in Schritt C) im Kreis geführten Kühlmittel und optional dem in Schritt D) im Kreis geführten Absorptionsmittel Proben entnommen werden und in den entnommenen Proben mittels lodometrie, Differential Scanning Calometrie (DSC) oder Mikrokalorimetrie der Peroxid-Gehalt bestimmt wird, wobei bei einer Zersetzungsenergie des im Kreis geführten Kühlmittels von größer 400 J/g Peroxide in dem Kühlmittel durch Erhitzen des Kühlmittels, Behandlung des Kühlmittels mit Basen, oder Hydrierung vernichtet werden.

Es wurde gefunden, dass sich die Bildung explosionsgefährlicher Peroxide in den flüssigen Prozessströmen erfindungsgemäß mittels kalorimetrischer Techniken wie der DSC oder Mikrokalorimetrie, beispielsweise mittels eines TAM-Mikrokalorimeters, überwachen lässt. Alternativ kann auch eine iodometrische Peroxid-Bestimmung eingesetzt werden, diese ist jedoch weniger genau. Bevorzugt werden daher kalorimetrische Techniken wie DSC und Mikrokalorimetrie eingesetzt. Mit den kalorimetrischen Techniken wird die Summe aller exothermen Reaktionen bei einer bestimmten Temperatur bestimmt, wodurch auf die maximal enthaltene Peroxid-Menge geschlossen werden kann. Die Berechnung der maximal in den Proben enthaltenen Peroxid-Menge geschieht aus der durch kalorimetrische Messung bestimmten Wärmemenge der exothermen Zersetzung in J/g und der bekannten Zersetzungsenergie der Peroxide von ca. 1340 J/g.

Bevorzugt wird der Peroxid-Gehalt in den entnommenen Proben mittels DSC bestimmt.

Die Proben können dem oder den Kühlmittelströmen und/oder dem oder den Absorptionsmittelströmen sowohl diskontinuierlich als auch kontinuierlich entnommen werden.

Die Dynamische Differenzkalorimetrie (DDK) (engl.: *Differential Scanning Calorimetry-*DSC) ist ein Verfahren der Thermischen Analyse zur Messung von abgegebener oder aufgenommener Wärmemenge einer Probe bei isothermer Arbeitsweise, Aufheizung oder Abkühlung. Ein verkapselter Behälter, Tiegel genannt, mit einer Probe und ein zweiter Behälter ohne Inhalt (Referenz) werden zusammen dem gleichen Temperaturprogramm ausgesetzt. Dabei kommt es infolge der Wärmekapazität der Probe und exothermer oder endothermer Prozesse zu Temperaturänderungen *ΔT* im Vergleich zur leeren Probe (*T*_{Ref}), da thermische Energie in den oder aus dem entsprechenden Prozess fließt.

Im Gegensatz zur älteren Differentialthermoanalyse (DTA) wird bei der DSC diese Temperaturdifferenz nicht direkt als Messsignal verwendet, sondern auf den Wärmestrom als Messgröße geschlossen. Dafür stehen zwei Verfahren zur Verfügung. Dynamische Wärmestromdifferenzkalorimetrie: Bei diesem auch *Heat flux DSC* genannten Typ werden die Enthalpieänderungen (Wärmestrom) durch Integration der*ΔT-T*_{Ref}-Kurve berechnet. Dabei befinden sich Stellflächen für Probe und Referenz im Ofen auf einer Scheibe (Disk type measuring system), welche eine gute Wärmeleitfähigkeit besitzt und unter welcher die Temperaturfühler sitzen. Wird der Ofen erhitzt, so fließt die Wärme durch die Probe/Referenz in die Scheibe und wird dort mittels der Fühler abgenommen. Sind Probe und Referenz gleich, fließen gleich große Wärmeströme durch die Scheibe. Die Wärmestromdifferenz ist damit null. Verändert sich während der Messung eine Probe, z. B. durch exotherme Zersetzung, entsteht eine Differenz im Wärmestrom, welche proportional zur Temperaturdifferenz ist.

Dynamische Leistungsdifferenzkalorimetrie: Bei diesem auch *Power compensating* DSC genannten Typ werden Probe und Referenztiegel in thermisch isolierte Öfen gebracht und diese so geregelt, dass auf beiden Seiten stets die gleiche Temperatur herrscht. Die dafür notwendige elektrische Leistung wird als Funktion der Temperatur aufgezeichnet.

Für mikrokalorimetrische Messungen kann beispielsweise das isothermal arbeitende Wärmeleitungsmikrokalorimeter Thermal Activity Monitor 2277 (TAM) des Herstellers Thermometric (Järfalla, Schweden) eingesetzt werden. Mit diesem Gerät ist es möglich, Temperaturdifferenzen von nur 10⁻⁶ °C zu erfassen, was einem Wärmestrom im µW-Bereich entspricht. Im Vergleich zur DSC ist durch eine bis zu ca. einhundertfach größere Probenmasse und eine bis zu ca. einhundertfach höhere Messempfindlichkeit die Gesamtempfindlichkeit um bis zu ca. den Faktor 10.000 erhöht.

Weitere Einzelheiten zur Dynamische Differenzkalorimetrie sind in W. F. Hemminger, H. K. Cammenga: Methoden der Thermischen Analyse. Springer-Verlag, ISBN 3-540-15049-8, und in G. Höhne, W. Hemminger, H.-J. Flammersheim: Differential Scanning Calorimetry - An introduction for Practioners. Springer-Verlag, Berlin 1996, beschrieben.

Als Kühlmittel wird in Schritt C) ein organisches Lösungsmittel verwendet. Bevorzugt sind aromatische KohlenwasserstoffLösungsmittel wie Toluol.

Nachstehende Ausführungsformen sind bevorzugte bzw. besonders bevorzugte Varianten des erfindungsgemäßen Verfahrens:
Die Stufe C) umfasst mindestens eine Abkühlungsstufe Ca) und eine Kompressionsstufe Cb). Die Stufe Ca) wird bevorzugt mehrstufig in Stufen Ca1) bis Can), insbesondere zweistufig in zwei Stufen Ca1) und Ca2) durchgeführt. Dabei wird in einer Variante zumindest ein Teil des Kühlmittels nach Durchlaufen der zweiten Stufe Ca2) als Kühlmittel der ersten Stufe Ca1) zugeführt.

Die Stufe Cb) umfasst im Allgemeinen mindestens eine Kompressionsstufe Cba) und mindestens eine Abkühlstufe Cbb). Bevorzugt wird in der mindestens einen Abkühlstufe Cbb) das in der Kompressionsstufe Cba) komprimierte Gas mit einem Kühlmittel in Kontakt gebracht. Besonders bevorzugt enthält das Kühlmittel der Abkühlstufe Cbb) das gleiche organische Lösungsmittel, das in der Stufe Ca) als Kühlmittel verwendet wird. In einer insbesonders bevorzugten Variante wird zumindest ein Teil dieses Kühlmittels nach Durchlaufen der mindestens einen Abkühlstufe Cbb) als Kühlmittel der Stufe Ca) zugeführt.

Bevorzugt umfasst die Stufe Cb) mehrere Kompressionsstufen Cba1) bis Cban) und Abkühlstufen Cbb1) bis Cbbn), beispielsweise vier Kompressionsstufen Cba1) bis Cba4) und vier Abkühlstufen Cbb1) bis Cbb4).

Erfindungsgemäß können einem, mehreren oder allen im Kreis geführten Kühlmittelströmen Proben entnommen werden, in denen erfindungsgemäß der Peroxidgehalt bestimmt wird.

Bevorzugt umfasst Schritt D) die Schritte Da) bis Dc):
Da) Absorption der C₄-Kohlenwasserstoffe umfassend Butadien und n-Butene in einem hochsiedenden Absorptionsmittel, wobei ein mit C₄-Kohlenwasserstoffen beladener Absorptionsmittelstrom und der Gasstrom d2 erhalten werden,
Db) Entfernung von Sauerstoff aus dem mit C₄-Kohlenwasserstoffen beladenen Absorptionsmittelstrom aus Schritt Da) durch Strippung mit einem nicht kondensierbaren Gasstrom, und
Dc) Desorption der C₄-Kohlenwasserstoffe aus dem beladenen Absorptionsmittelstrom, wobei ein C₄-Produktgasstrom d1 erhalten wird. Dieser weist dann bevorzugt einen Sauerstoffgehalt von weniger als 100 ppm auf.

In einer Ausführungsform ist das in Schritt D) verwendete Absorptionsmittel das gleiche organische Lösungsmittel wie ein in Schritt C) verwendetes Kühlmittel, wobei zumindest ein Teil dieses Absorptionsmittels nach Desorption der C₄-Kohlenwasserstoffe als Kühlmittel dem Schritt C) zugeführt wird. In einer bevorzugten Variante dieser Ausführungsform ist das Absorptions- und Kühlmittel Toluol.

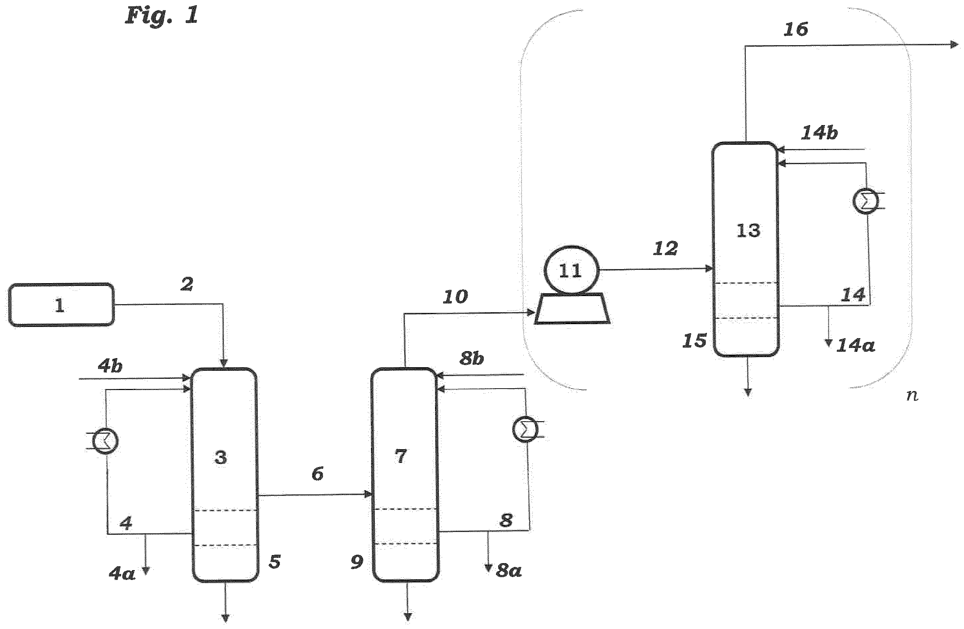

Bevorzugte Ausführungsformen des Verfahrens sind in den Figuren 1-3 dargestellt und werden im Folgenden im Einzelnen beschrieben.

In einem Schritt A) wird ein n-Butene enthaltender Einsatzgasstrom bereitgestellt.

Als Einsatzgasstrom können reine n-Butene (1-Buten und/oder cis-/trans-2-Buten), aber auch Butene enthaltende Gasgemische eingesetzt werden. Ein solches Gasgemisch kann beispielsweise durch nicht-oxidative Dehydrierung von n-Butan erhalten werden. Es kann auch eine Fraktion eingesetzt werden, die als Hauptbestandteil n-Butene (1-Buten und cis-/trans-2-Buten) enthält und aus der C₄-Fraktion des Naphtha-Crackens durch Abtrennung von Butadien und iso-Buten erhalten wurde. Des Weiteren können auch Gasgemische als Ausgangsgas eingesetzt werden, die reines 1-Buten, cis-2-Buten, trans-2-Buten oder Mischungen daraus umfassen, und die durch Dimerisierung von Ethylen erhalten wurden. Ferner können als Ausgangsgas n-Butene enthaltende Gasgemische eingesetzt werden, die durch katalytisches Wirbelschichtkracken (Fluid Catalytic Cracking, FCC) erhalten wurden.

In einer Ausführungsform des erfindungsgemäßen Verfahrens wird das n-Butene enthaltende Ausgangsgasgemisch durch nicht-oxidative Dehydrierung von n-Butan erhalten. Durch die Kopplung einer nicht-oxidativen katalytischen Dehydrierung mit der oxidativen Dehydrierung der gebildeten n-Butene kann eine hohe Ausbeute an Butadien, bezogen auf eingesetztes n-Butan, erhalten werden. Bei der nicht-oxidativen katalytischen n-Butan-Dehydrierung wird ein Gasgemisch erhalten, das neben Butadien 1-Buten, 2-Buten und nicht umgesetztem n-Butan Nebenbestandteile enthält. Übliche Nebenbestandteile sind Wasserstoff, Wasserdampf, Stickstoff, CO und CO₂, Methan, Ethan, Ethen, Propan und Propen. Die Zusammensetzung des die erste Dehydrierzone verlassenden Gasgemischs kann abhängig von der Fahrweise der Dehydrierung stark variieren. So weist bei Durchführung der Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

In Schritt B) werden der n-Butene enthaltende Einsatzgasstrom und ein sauerstoffhaltiges Gas in mindestens eine Dehydrierzone 1 (ODH-Reaktor) eingespeist und die in dem Gasgemisch enthaltenen Butene in Gegenwart eines Oxidehydrierungskatalysators oxidativ zu Butadien dehydriert.

Für die Oxidehydrierung geeignete Katalysatoren basieren im Allgemeinen auf einem Mo-Bi-O-haltigen Multimetalloxidsystem, das in der Regel zusätzlich Eisen enthält. Im Allgemeinen enthält das Katalysatorsystem noch weitere zusätzliche Komponenten, wie beispielsweise Kalium, Cäsium, Magnesium, Zirkon, Chrom, Nickel, Cobalt, Cadmium, Zinn, Blei, Germanium, Lanthan, Mangan, Wolfram, Phosphor, Cer, Aluminium oder Silizium. Auch eisenhaltige Ferrite wurden als Katalysatoren vorgeschlagen.

In einer bevorzugten Ausführungsform enthält das Multimetalloxid Cobalt und/oder Nickel. In einer weiteren bevorzugten Ausführungsform enthält das Multimetalloxid Chrom. In einer weiteren bevorzugten Ausführungsform enthält das Multimetalloxid Mangan.

Beispiele für Mo-Bi-Fe-O-haltige Multimetalloxide sind Mo-Bi-Fe-Cr-O- oder Mo-Bi-Fe-Zr-O-haltige Multimetalloxide. Bevorzugte Systeme sind beispielsweise beschrieben in US 4,547,615 (Mo₁₂BiFe_{0,1}Ni₈ZrCr₃K₀,₂Oₓ und Mo₁₂BiFe_{0,1}Ni₈AlCr₃K_{0,2}Oₓ), US 4,424,141 (Mo₁₂BiFe₃Co_{4,5}Ni_{2,5}P_{0,5}K_{0,1}Oₓ + SiO₂), DE-A 25 30 959 (Mo₁₂BiFe₃Co_{4,5}Ni_{2,5}Cr_{0,5}K_{0,1}Oₓ, Mo_{13,75}BiFe₃Co_{4,5}Ni_{2,5}Ge_{0,5}K_{0,8}Oₓ, Mo₁₂BiFe₃Co_{4,5}Ni_{2,5}Mn_{0,5}K_{0,1}Oₓ und Mo₁₂BiFe₃Co_{4,5}Ni_{2,5}La_{0,5}K_{0,1}Oₓ), US 3,911,039 (Mo₁₂BiFe₃Co_{4,5}Ni_{2,5}Sn_{0,5}K_{0,1}Oₓ), DE-A 25 30 959 und DE-A 24 47 825 (Mo₁₂BiFe₃Co_{4,5}Ni_{2,5}W_{0,5}K_{0,1}Oₓ).

Geeignete Multimetalloxide und deren Herstellung sind weiterhin beschrieben in US 4,423,281 (Mo₁₂BiNi₈Pb_{0,5}Cr₃K_{0,2}Oₓ und Mo₁₂Bi_{b}Ni₇Al₃Cr_{0,5}K_{0,5}Oₓ), US 4,336,409 (Mo₁₂BiNi₆Cd₂Cr₃P_{0,5}Oₓ), DE-A 26 00 128 (Mo₁₂BiNi_{0,5}Cr₃P_{0,5}Mg_{7,5}K_{0,1}Oₓ + SiO₂) und DE-A 24 40 329 (Mo₁₂BiCo_{4,5}Ni_{2,5}Cr₃P_{0,5}K_{0,1}Oₓ).

Besonders bevorzugte katalytisch aktive, Molybdän und mindestens ein weiteres Metall enthaltende Multimetalloxide weisen die allgemeine Formel (Ia) auf:

Mo₁₂BiₐFe_{b}CO_{c}Ni_{d}CrₑX¹_{f}X²_{g}O_{y} (Ia),

mit
X¹ = Si, Mn und/oder Al,
X²= Li, Na, K, Cs und/oder Rb,
0,2 ≤a ≤ 1,
0,5 ≤ b ≤ 10,
0 ≤ c ≤ 10,
0 ≤ d ≤ 10,
2 ≤ c + d ≤ 10
0 ≤ e ≤ 2,
0 ≤ f ≤ 10
0 ≤ g ≤ 0,5
y = eine Zahl, die unter der Voraussetzung der Ladungsneutralität durch die Wertigkeit und Häufigkeit der von Sauerstoff verschiedenen Elemente in (la) bestimmt wird.

Bevorzugt sind Katalysatoren, deren katalytisch aktive Oxidmasse von den beiden Metallen Co und Ni nur Co aufweist (d = 0). Bevorzugte ist X¹ Si und/oder Mn und X² ist vorzugsweise K, Na und/oder Cs, besonders bevorzugt ist X² = K.

Das molekularen Sauerstoff enthaltende Gas enthält im Allgemeinen mehr als 10 Vol.-%, vorzugsweise mehr als 15 Vol.-% und noch mehr bevorzugt mehr als 20 Vol.-% molekularen Sauerstoff. Bevorzugt ist es Luft. Die Obergrenze für den Gehalt an molekularem Sauerstoff beträgt im Allgemeinen 50 Vol.-% oder weniger, vorzugsweise 30 Vol.-% oder weniger und noch mehr bevorzugt 25 Vol.-% oder weniger. Darüber hinaus können in dem molekularen Sauerstoff enthaltenden Gas beliebige Inertgase enthalten sein. Als mögliche Inertgase können Stickstoff, Argon, Neon, Helium, CO, CO₂ und Wasser genannt werden. Die Menge an Inertgasen beträgt für Stickstoff im Allgemeinen 90 Vol.-% oder weniger, vorzugsweise 85 Vol.-% oder weniger und noch mehr bevorzugt 80 Vol.-% oder weniger. Im Falle anderer Bestandteile als Stickstoff beträgt sie im Allgemeinen 10 Vol.-% oder weniger, vorzugsweise 1 Vol.-% oder weniger.

Zur Durchführung der oxidativen Dehydrierung bei Vollumsatz von n-Butenen ist ein Gasgemisch bevorzugt, welches ein molares Sauerstoff: n-Butene-Verhältnis von mindestens 0,5 aufweist. Bevorzugt wird bei einem Sauerstoff: n-Butene-Verhältnis von 0,55 bis 10 gearbeitet. Zur Einstellung dieses Wertes kann das Ausgangsstoffgas mit Sauerstoff oder einem sauerstoffhaltigem Gas, beispielsweise Luft, und gegebenenfalls zusätzlichem Inertgas oder Wasserdampf vermischt werden. Das erhaltene sauerstoffhaltige Gasgemisch wird dann der Oxidehydrierung zugeführt.

Die Reaktionstemperatur der Oxidehydrierung wird im Allgemeinen durch ein Wärmeaustauschmittel, welches sich um die Reaktionsrohre herum befindet, kontrolliert. Als solche flüssige Wärmeaustauschmittel kommen z. B. Schmelzen von Salzen wie Kaliumnitrat, Kaliumnitrit, Natriumnitrit und/oder Natriumnitrat sowie Schmelzen von Metallen wie Natrium, Quecksilber und Legierungen verschiedener Metalle in Betracht. Aber auch ionische Flüssigkeiten oder Wärmeträgeröle sind einsetzbar. Die Temperatur des Wärmeaustauschmittels liegt zwischen 220 bis 490 °C und bevorzugt zwischen 300 bis 450 °C und besonders bevorzugt zwischen 350 und 420 °C.

Auf Grund der Exothermie der ablaufenden Reaktionen kann die Temperatur in bestimmten Abschnitten des Reaktorinneren während der Reaktion höher liegen als diejenige des Wärmeaustauschmittels und es bildet sich ein so genannter Hotspot aus. Die Lage und Höhe des Hotspots ist durch die Reaktionsbedingungen festgelegt, aber sie kann auch durch das Verdünnungsverhältnis der Katalysatorschicht oder den Durchfluss an Mischgas reguliert werden. Die Differenz zwischen Hotspot-Temperatur und der Temperatur des Wärmeaustauschmittels liegt im Allgemeinen zwischen 1-150 °C, bevorzugt zwischen 10-100 °C und besonders bevorzugt zwischen 20-80 °C. Die Temperatur am Ende des Katalysatorbettes liegt im Allgemeinen zwischen 0-100 °C, vorzugsweise zwischen 0,1-50 °C, besonders bevorzugt zwischen 1-25 °C oberhalb der Temperatur des Wärmeaustauschmittels.

Die Oxidehydrierung kann in allen aus dem Stand der Technik bekannten Festbettreaktoren durchgeführt werden, wie beispielsweise im Hordenofen, im Festbettrohr- oder Rohrbündelreaktor oder im Plattenwärmetauscherreaktor. Ein Rohrbündelreaktor ist bevorzugt.

Vorzugsweise wird die oxidative Dehydrierung in Festbettrohrreaktoren oder Festbettrohrbündelreaktoren durchgeführt. Die Reaktionsrohre werden (ebenso wie die anderen Elemente des Rohrbündelreaktors) in der Regel aus Stahl gefertigt. Die Wanddicke der Reaktionsrohre beträgt typischerweise 1 bis 3 mm. Ihr Innendurchmesser liegt in der Regel (einheitlich) bei 10 bis 50 mm oder bei 15 bis 40 mm, häufig bei 20 bis 30 mm. Die im Rohrbündelreaktor untergebrachte Anzahl an Reaktionsrohren beläuft sich in der Regel wenigstens auf 1000, oder 3000, oder 5000, vorzugsweise auf wenigstens 10 000. Häufig beträgt die Anzahl der im Rohrbündelreaktor untergebrachten Reaktionsrohre 15 000 bis 30 000 bzw. bis 40 000 oder bis 50 000. Die Länge der Reaktionsrohre erstreckt sich im Normalfall auf wenige Meter, typisch ist eine Reaktionsrohrlänge im Bereich von 1 bis 8 m, häufig 2 bis 7 m, vielfach 2,5 bis 6 m.

Weiterhin kann die Katalysatorschicht, die im Reaktor **1** eingerichtet ist, aus einer einzelnen Schicht oder aus 2 oder mehr Schichten bestehen. Diese Schichten können aus reinem Katalysator bestehen oder mit einem Material verdünnt sein, das nicht mit dem Ausgangsgas oder Komponenten aus dem Produktgas der Reaktion reagiert. Weiterhin können die Katalysatorschichten aus Vollmaterial oder geträgerten Schalenkatalysatoren bestehen.

Der die oxidative Dehydrierung verlassende Produktgasstrom **2** enthält neben Butadien im Allgemeinen noch nicht umgesetztes 1-Buten und 2-Buten, Sauerstoff sowie Wasserdampf. Als Nebenkomponenten enthält er weiterhin im Allgemeinen Kohlenmonoxid, Kohlendioxid, Inertgase (hauptsächlich Stickstoff), leichtsiedende Kohlenwasserstoffe wie Methan, Ethan, Ethen, Propan und Propen, Butan und iso-Butan, gegebenenfalls Wasserstoff sowie gegebenenfalls sauerstoffhaltige Kohlenwasserstoffe, sogenannte Oxygenate. Oxygenate können beispielsweise Formaldehyd, Furan, Essigsäure, Maleinsäureanhydrid, Ameisensäure, Methacrolein, Methacrylsäure, Crotonaldehyd, Crotonsäure, Propionsäure, Acrylsäure, Methylvinylketon, Styrol, Benzaldehyd, Benzoesäure, Phthalsäureanhydrid, Fluorenon, Anthrachinon und Butyraldehyd sein.

Der Produktgasstrom **2** am Reaktorausgang ist durch eine Temperatur nahe der Temperatur am Ende des Katalysatorbetts charakterisiert. Der Produktgasstrom wird dann auf eine Temperatur von 150 - 400 °C, bevorzugt 160 - 300 °C, besonders bevorzugt 170 - 250 °C gebracht. Es ist möglich, die Leitung, durch die der Produktgasstrom fließt, um die Temperatur im gewünschten Bereich zu halten, zu isolieren, jedoch ist ein Einsatz eines Wärmetauschers bevorzugt. Dieses Wärmetauschersystem ist beliebig, solange mit diesem System die Temperatur des Produktgases auf dem gewünschten Niveau gehalten werden kann. Als Beispiel eines Wärmetauschers können Spiralwärmetauscher, Plattenwärmetauscher, Doppelrohrwärmetauscher, Multirohrwärmetauscher, Kessel-Spiralwärmetauscher, Kessel-Mantelwärmetauscher, Flüssigkeit-Flüssigkeit-Kontakt-Wärmetauscher, Luft-Wärmetauscher, Direktkontaktwärmetauscher sowie Rippenrohrwärmetauscher genannt werden. Da, während die Temperatur des Produktgases auf die gewünschte Temperatur eingestellt wird, ein Teil der hochsiedenden Nebenprodukte, die im Produktgas enthalten sind, ausfallen kann, sollte daher das Wärmetauschersystem vorzugsweise zwei oder mehr Wärmetauscher aufweisen. Falls dabei zwei oder mehr vorgesehene Wärmetauscher parallel angeordnet sind, und so eine verteilte Kühlung des gewonnenen Produktgases in den Wärmetauschern ermöglicht wird, nimmt die Menge an hochsiedenden Nebenprodukten, die sich in den Wärmetauschern ablagern, ab und so kann ihre Betriebsdauer verlängert werden. Als Alternative zu der oben genannten Methode können die zwei oder mehr vorgesehenen Wärmetauscher parallel angeordnet sein. Das Produktgas wird einem oder mehreren, nicht aber allen, Wärmetauschern zugeführt, welche nach einer gewissen Betriebsdauer von anderen Wärmetauschern abgelöst werden. Bei dieser Methode kann die Kühlung fortgesetzt werden, ein Teil der Reaktionswärme zurückgewonnen und parallel dazu können die in einem der Wärmetauscher abgelagerten hochsiedenden Nebenprodukte entfernt werden. Als ein oben genanntes organisches Lösungsmittel kann ein Lösungsmittel, solange es in der Lage ist, die hochsiedenden Nebenprodukte aufzulösen, uneingeschränkt verwendet werden, und als Beispiele dazu können ein aromatisches Kohlenwasserstofflösungsmittel, wie z. B. Toluol, Xylen etc. sowie ein alkalisches wässriges Lösungsmittel, wie z. B. die wässrige Lösung von Natriumhydroxid, verwendet werden.

Anschließend können im Schritt C) aus dem Produktgasstrom **2** durch Abkühlung ein Großteil der hochsiedenden Nebenkomponenten und des Wassers abgetrennt werden. Diese Abkühlung und Abtrennung erfolgt dabei vorzugsweise in einem Quench. Dieser Quench kann aus einer (**3** in Figur 1) oder mehreren Stufen bestehen (**3, 7** in Figur 1.). Vorzugsweise wird ein Verfahren eingesetzt, bei dem der Produktgasstrom **2** direkt mit dem Kühlmittel **4** in Kontakt gebracht und dadurch gekühlt wird. Als Kühlmittel können Wasser, alkalische wässrige Lösungen, organische Lösungsmittel oder eine Kombination oder Gemisch daraus verwendet werden. Vorzugsweise wird ein organisches Lösungsmittel verwendet. Als Lösungsmittel kann, solange es in der Lage ist, Teile der im Gasstrom befindlichen Nebenkomponenten aufzunehmen, jedes Lösungsmittel verwendet werden. Besonders bevorzugt sind Lösungsmittel wie Toluol und Ketone.

In einer besonders bevorzugten Ausführungsform des erfindungsgemäßen Verfahrens wird Toluol als Kühlmittel verwendet.

Bevorzugt ist ein zweistufiger Quench. Die Kühlungstemperatur des Produktgases unterscheidet sich je nach Art der Temperatur des aus dem Reaktorauslass erhaltenen Produktgases **2** und des Kühlmittels **4.** Im Allgemeinen hat das Produktgas **2** je nach Vorliegen und Temperaturniveau eines Wärmetauschers vor dem Quencheingang eine Temperatur von 100-440 °C. Der Produktgaseinlass in den Quench muss so ausgelegt sein, dass ein Verstopfen durch Ablagerungen am und direkt vor dem Gaseinlass minimiert oder verhindert wird. Das Produktgas wird in der 1. Quenchstufe **3** mit dem Kühlmittel in Kontakt gebracht. Hierbei kann das Kühlmittel durch eine Düse eingebracht werden, um eine möglichst effiziente Durchmischung mit dem Produktgas zu erreichen. Zum gleichen Zweck können in der Quenchstufe Einbauten, wie zum Beispiel weitere Düsen, eingebracht werden, durch die das Produktgas und das Kühlmittel gemeinsam passieren müssen. Der Kühlmitteleinlass in den Quench muss so ausgelegt sein, dass ein Verstopfen durch Ablagerungen im Bereich des Kühlmitteleinlasses minimiert oder verhindert wird.

Im Allgemeinen wird das Produktgas **2** in der ersten Quenchstufe auf 5-180 °C, vorzugsweise auf 30-130 °C und noch mehr bevorzugt auf 60-110 °C gekühlt. Die Temperatur des Kühlmittelmediums **4** am Einlass kann im Allgemeinen 25-200 °C, bevorzugt 40-120 °C, insbesondere bevorzugt 50-90 °C betragen. Der Druck in der ersten Quenchstufe ist nicht besonders eingeschränkt, beträgt aber im Allgemeinen 0,01-4 bar (ü), bevorzugt 0.1-2 bar (ü) und besonders bevorzugt 0.2-1 bar (ü). Wenn viel hochsiedende Nebenprodukte im Produktgas vorhanden sind, kommt es leicht zu Polymerisation der hochsiedenden Nebenprodukte und zu Ablagerungen von Feststoffen, die durch hochsiedende Nebenprodukte in diesem Verfahrensabschnitt verursacht werden. Das im Kühlturm der ersten Quenchstufe eingesetzt Kühlmittel **4** wird zirkulierend eingesetzt. Der Kreislaufstrom des Kühlmittels in Liter pro Stunde bezogen auf den Massenstrom an Butadien in Gramm pro Stunde kann im Allgemeinen 0.0001-5 l/g, bevorzugt 0.001-1 l/g und besonders bevorzugt 0.002-0.2 l/g betragen.

Die Temperatur des Kühlmittels **4** im Sumpf kann im Allgemeinen 27-210 °C, bevorzugt 45-130 °C, insbesondere bevorzugt 55-95 °C betragen. Da die Beladung des Kühlmittels **4** mit Nebenkomponenten im Laufe der Zeit zunimmt, kann ein Teil des beladenen Kühlmittels aus dem Umlauf abgezogen werden (**4a**) und die Umlaufmenge durch Zugabe von unbeladenem Kühlmittel (**4b**) konstant gehalten werden. Das Verhältnis von Ablaufmenge und Zugabemenge hängt von der Dampfbeladung des Produktgases und der Produktgastemperatur am Ende der erste Quenchstufe ab. Je nach Temperatur, Druck und Wassergehalt des Produktgases 2 kann es in der ersten Quenchstufe **3** zur Kondensation von Wasser kommen. In diesem Falle kann sich eine zusätzliche wässrige Phase **5** bilden, welche zusätzlich wasserlösliche Nebenkomponenten enthalten kann. Diese kann dann im Sumpf der Quenchstufe **3** abgezogen werden. Bevorzugt ist ein Betrieb, in dem sich in der ersten Quenchstufe **3** keine wässrige Phase ausbildet. Der abgekühlte und eventuell an Nebenkomponenten abgereicherte Produktgasstrom **6** kann nun einer zweiten Quenchstufe **7** zugeführt werden. In dieser kann er nun erneut mit einem Kühlmittel **8** in Kontakt gebracht werden.

Als Kühlmittel **8** können Wasser, alkalische wässrige Lösungen, organische Lösungsmittel oder eine Kombination oder Gemische daraus verwendet werden. Vorzugsweise wird ein organisches Lösungsmittel verwendet. Als ein oben genanntes Lösungsmittel kann, solange es in der Lage ist, Teile der im Gasstrom befindlichen Nebenkomponenten aufzunehmen, jedes Lösungsmittel verwendet werden. Bevorzugt sind aromatische Kohlenwasserstoff- Lösungsmittel wie Toluol, da in diesen die Löslichkeitsgrenze größer 1000 ppm mg aktiver Sauerstoff / kg Lösungsmittel ist.

In einer Ausführungsform der Erfindung wird die Peroxid-Konzentration in den Strömen **4** und **8** bestimmt.

Falls eine kritische Konzentration von Peroxiden erreicht ist, welche einer Zersetzungsenergie des im Kreis geführten Kühlmittels von größer 400 J/g, bevorzugt aber von größer 100 J/g entspricht, werden erfindungsgemäß Maßnahmen zur Reduzierung der Per-oxidmenge in dem Kühlmittel durchgeführt. Diese Maßnahmen sind ausgewählt aus einer Peroxid-Zerstörungen durch Anwendung erhöhter Temperaturen, wie von Hendry 1968 (luEC Product Research and Development, Vol.7,No.2, 1968, 136-145) beschrieben, Behandlung mit starken Basen, wie ebenfalls von Hendry 1968 (luEC Product Research and Development, Vol.7,No.2, 1968, 145-151) beschrieben, oder einer Hydrierung an heterogenen Katalysatoren. Hierzu kann das die Peroxide enthaltende Kühlmittel erhitzt oder das Kühlmittel kann, beispielsweise in einem Rührbehälter, mit einer basischen wässrigen Lösung innig vermischt werden, wobei die Phasen anschließend wieder getrennt werden, oder aber die in dem Kühlmittel enthaltenen Peroxide werden durch Hydrierung an einem heterogenen Festbettkatalysator entfernt.

Im Allgemeinen wird das Produktgas bis zum Gasausgang der zweiten Quenchstufe **7** auf 5 bis 100 °C, vorzugsweise auf 15-85 °C und noch mehr bevorzugt auf 30-70 °C gekühlt. Das Kühlmittel kann im Gegenstrom zum Produktgas zugeführt werden. In diesem Fall kann die Temperatur des Kühlmittelmediums **8** am Kühlmitteleinlass 5-100 °C, bevorzugt 15-85 °C, insbesondere bevorzugt 30-70 °C betragen. Der Druck in der zweiten Quenchstufe **7** ist nicht besonders eingeschränkt, beträgt aber im Allgemeinen 0,01-4 bar (ü), bevorzugt 0,1-2 bar (ü) und besonders bevorzugt 0,2-1 bar (ü). Das im Kühlturm der zweiten Quenchstife eingesetzte Kühlmittel 8 wird zirkulierend eingesetzt. Der Kreislaufstrom des Kühlmittels **8** in Liter pro Stunde bezogen auf den Massenstrom an Butadien in Gramm pro Stunde kann im Allgemeinen 0,0001-5 l/g, bevorzugt 0,3001-1 l/g und besonders bevorzugt 0,002-0.2 l/g betragen.

Je nach Temperatur, Druck und Wassergehalt des Produktgases **6** kann es in der zweiten Quenchstufe **7** zur Kondensation von Wasser kommen. In diesem Falle kann sich eine zusätzliche wässrige Phase **9** bilden, welche zusätzlich wasserlösliche Nebenkomponenten enthalten kann. Diese kann dann im Sumpf der Quenchstufe **7** abgezogen werden. Befindet sich im Sumpf der zweiten Qenchstufe **7** eine wässrige Phase **9** oder wenn in einem Teil des Quenches Wasser als Kühlmittel eingesetzt wird, gelingt das Einlösen von Nebenprodukten der ODH-Reaktion, zum Beispiel Essigsäure, MSA, etc. bei erhöhtem pH-Wert besser als bei niedrigem pH-Wert. Da das Einlösen von Nebenprodukten wie den oben genannten den pH-Wert von zum Beispiel Wasser erniedrigt, kann der pH-Wert durch Zugabe eines alkalischen Mediums konstant gehalten oder erhöht werden. Im Allgemeinen wird der pH-Wert der wässrigen Phase im Sumpf der zweiten Quenchstufe **7** zwischen 1-14, bevorzugt zwischen 2-12, besonders bevorzugt zwischen 3-11 gehalten. Je saurer der Wert, desto weniger alkalisches Medium muss zugeführt werden. Je basischer, desto besser gelingt die Einlösung einiger Nebenprodukte. Jedoch führen sehr hohe pH-Werte zum Einlösen von Nebenprodukten wie CO₂ und damit zu einem sehr hohen Verbrauch des alkalischen Mediums. Die Temperatur des Kühlmittels **8** im Sumpf kann im Allgemeinen 20-210 °C, bevorzugt 35-120 °C, insbesondere bevorzugt 45-85 °C betragen. Da die Beladung des Kühlmittels **8** mit Nebenkomponenten im Laufe der Zeit zunimmt, kann ein Teil des beladenen Kühlmittels aus dem Umlauf abgezogen werden (**8a**) und die Umlaufmenge durch Zugabe von unbeladenem Kühlmittel (**8b**) konstant gehalten werden.

Um einen möglichst guten Kontakt von Produktgas und Kühlmittel zu erreichen, können Einbauten in der zweiten Quenchstufe vorhanden sein. Solche Einbauten umfassen zum Beispiel Glocken-, Zentrifugal- und/oder Siebböden, Kolonnen mit strukturierten Packungen, z. B. Blechpackungen mit einer spezifischen Oberfläche von 100 bis 1000 m²/m³ wie Mellapak® 250 Y, und Füllkörperkolonnen.

Die Umläufe der beiden Quenchstufen können sowohl voneinander getrennt als auch miteinander verbunden sein. So kann beispielsweise der Strom **8a** dem Strom **4b** zugeführt werden oder diesen ersetzen. Die gewünschte Temperatur der Umlaufströme kann über geeignete Wärmetauscher eingestellt werden.

Um den Mitriss von flüssigen Bestandteilen aus dem Quench in die Abgasleitung zu minimieren, können geeignete bauliche Maßnahmen, wie zum Beispiel der Einbau eines Demisters, getroffen werden. Weiterhin können hochsiedende Substanzen, welche im Quench nicht vom Produktgas abgetrennt werden durch weitere bauliche Maßnahmen, wie beispielsweise weitere Gaswäschen, aus dem Produktgas entfernt werden. Es wird ein Gasstrom **10** erhalten, in welchem n-Butan, 1-Buten, 2-Butene, Butadien, gegebenenfalls Sauerstoff, Wasserstoff, Wasserdampf, in geringen Mengen Methan, Ethan, Ethen, Propan und Propen, iso-Butan, Kohlenstoffoxide, Inertgase und Teile des im Quench verwendeten Lösungsmittel verbleiben. Weiterhin können in diesem Produktgasstrom Spuren von hochsiedenden Komponenten verbleiben, welche im Quench nicht quantitativ abgetrennt wurden.

Anschließend wird der Produktgasstrom **10** aus dem Quench in mindestens einer Kompressionsstufe **11** komprimiert und nachfolgend in der Kühlstufe **13** weiter abgekühlt, wobei mindestens ein Kondensatstrom enthaltend Wasser **15** und das im Quench verwendete Lösungsmittel **14** auskondensieren, und ein Gasstrom16 enthaltend Butadien, 1-Buten, 2-Butene, Sauerstoff, Wasserdampf, gegebenenfalls leichtsiedende Kohlenwasserstoffe wie Methan, Ethan, Ethen, Propan und Propen, Butan und iso-Butan, gegebenenfalls Kohlenstoffoxide und gegebenenfalls Inertgase verbleiben. Weiterhin können in diesem Produktgasstrom Spuren von hochsiedenden Komponenten verbleiben.

Die Kompression und Kühlung des Gasstroms **10** kann ein- oder mehrstufig (n-stufig) erfolgen. Im Allgemeinen wird insgesamt von einem Druck im Bereich von 1,0 bis 4,0 bar (absolut) auf einen Druck im Bereich von 3,5 bis 20 bar (absolut) komprimiert. Nach jeder Kompressionsstufe folgt eine Abkühlstufe, in der der Gasstrom auf eine Temperatur im Bereich von 15 bis 60 °C abgekühlt wird. Der Kondensatstrom kann somit bei mehrstufiger Kompression auch mehrere Ströme umfassen. Der Kondensatstrom besteht zu großen Teilen aus Wasser **15** und dem im Quench verwendeten Lösungsmittel **16**. Beide Ströme können daneben in geringem Umfang Leichtsieder, C₄-Kohlenwasserstoffe, Oxygenate und Kohlenstoffoxide enthalten.

Um den Strom **12** zu kühlen und/oder um weitere Nebenkomponenten aus dem Strom **12** zu entfernen, kann das kondensierte, im Quench verwendete Lösungsmittel **14** in einem Wärmetauscher abgekühlt und in die Kühlstufe **13** rückgeführt werden. Da die Beladung des Kühlmittels **14** mit Nebenkomponenten im Laufe der Zeit zunimmt, kann ein Teil des beladenen Kühlmittels **14a** aus dem Umlauf abgezogen werden und die Umlaufmenge durch Zugabe von unbeladenem Kühlmittel **14b** konstant gehalten werden.

Der Kondensatstrom **14a** kann in den Kreislaufstrom **4b** und/oder **8b** des Quenches zurückgeführt werden. Dadurch können die im Kondensatstrom **14a** absorbierten C₄-Komponenten wieder in den Gasstrom gebracht und damit die Ausbeute erhöht werden.

In einer Ausführungsform wird die Peroxid-Konzentration in Strom **14** bestimmt. In einer bevorzugten Ausführungsform wird die Peroxid-Konzentration in den Strömen **4, 8** und **14** bestimmt.

Geeignete Verdichter sind beispielsweise Turbo-, Drehkolben- und Hubkolbenverdichter. Die Verdichter können beispielsweise mit einem Elektromotor, einem Expander oder einer Gasoder Dampfturbine angetrieben werden. Typische Verdichtungsverhältnisse (Austrittsdruck : Eintrittsdruck) pro Verdichterstufe liegen je nach Bauart zwischen 1,5 und 3,0. Die Abkühlung des verdichteten Gases erfolgt mit Wärmetauschern, die beispielsweise als Rohrbündel-, Spiral- oder Plattenwärmetauscher ausgeführt sein können. Als Kühlmittel kommen in den Wärmetauschern dabei Kühlwasser oder Wärmeträgeröle zum Einsatz. Daneben wird bevorzugt Luftkühlung unter Einsatz von Gebläsen eingesetzt.

Der Butadien, n-Butene, Sauerstoff, leicht siedende Kohlenwasserstoffe (Methan, Ethan, Ethen, Propan, Propen, n-Butan, iso-Butan), gegebenenfalls Wasserdampf, gegebenenfalls Kohlenstoffoxide sowie gegebenenfalls Inertgase enthaltende Gasstrom **16** wird als Ausgangsstrom der weiteren Aufbereitung zugeführt.

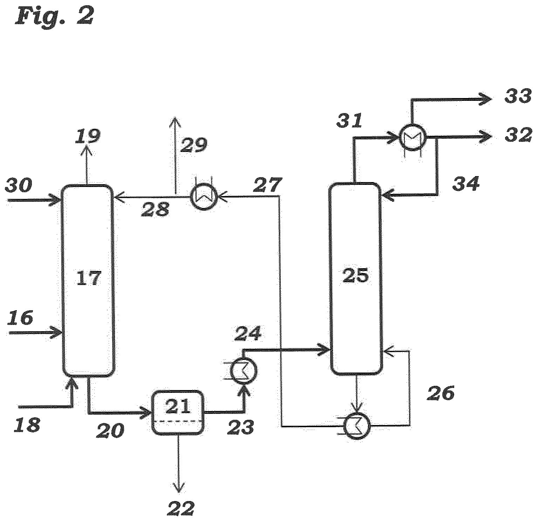

In einem Schritt D) (Figur 2) werden nicht kondensierbare und leicht siedenden Gasbestandteile, umfassend Sauerstoff, leicht siedenden Kohlenwasserstoffe (Methan, Ethan, Ethen, Propan, Propen), Kohlenstoffoxide und Inertgase in einer Absorptionskolonne **17** als Gasstrom **19** aus dem Prozessgasstrom **16** durch Absorption der C₄-Kohlenwasserstoffe in einem hochsiedenden Absorptionsmittel (**28 und/oder 30**) und nachfolgender Desorption der C₄-Kohlenwasserstoffe abgetrennt. Dieser Schritt D) umfasst bevorzugt die Teilschritte
Da) Absorption der C₄-Kohlenwasserstoffe umfassend Butadien und n-Butene in einem hochsiedenden Absorptionsmittel (**28 und/oder 30**), wobei ein mit C₄-Kohlenwasserstoffen beladener Absorptionsmittelstrom und der Gasstrom **19** erhalten werden,
Db) Entfernung von Sauerstoff aus dem mit C₄-Kohlenwasserstoffen beladenen Absorptionsmittelstrom aus Schritt Da) durch Strippung mit einem nicht kondensierbaren Gasstrom **18** wobei ein mit C₄-Kohlenwasserstoffen beladener Absorptionsmittelstrom **20** erhalten wird und
Dc) Desorption der C₄-Kohlenwasserstoffe aus dem beladenen Absorptionsmittelstrom, wobei ein C₄-Produktgasstrom **31** erhalten wird.

Dazu wird in der Absorptionsstufe **17** der Gasstrom **16** mit einem inerten Absorptionsmittel in Kontakt gebracht und werden die C₄-Kohlenwasserstoffe in dem inerten Absorptionsmittel absorbiert, wobei ein mit C₄-Kohlenwasserstoffen beladenes Absorptionsmittel **20** und ein die übrigen Gasbestandteile enthaltendes Abgas **19** erhalten werden. In einer Desorptionsstufe werden die C₄-Kohlenwasserstoffe aus dem hochsiedenden Absorptionsmittel wieder freigesetzt.

Die Absorptionsstufe kann in jeder beliebigen, dem Fachmann bekannten geeigneten Absorptionskolonne durchgeführt werden. Die Absorption kann durch einfaches Durchleiten des Produktgasstroms durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Bevorzugt wird die Absorption im Gegenstrom durchgeführt. Geeignete Absorptionskolonnen sind z. B. Bodenkolonnen mit Glocken-, Zentrifugal- und/oder Siebboden, Kolonnen mit strukturierten Packungen, z. B. Blechpackungen mit einer spezifischen Oberfläche von 100 bis 1000 m²/m³ wie Mellapak® 250 Y, und Füllkörperkolonnen. Es kommen aber auch Riesel- und Sprühtürme, Graphitblockabsorber, Oberflächenabsorber wie Dickschicht und Dünnschichtabsorber sowie Rotationskolonnen, Tellerwäscher, Kreuzschleierwäscher und Rotationswäscher in Betracht.

In einer Ausführungsform wird einer Absorptionskolonne im unteren Bereich der Butadien, n-Butene und die leichtsiedenden und nicht kondensierbaren Gasbestandteile enthaltende Gasstrom**16** zugeführt. Im oberen Bereich der Absorptionskolonne wird das hochsiedende Absorptionsmittel (**28 und/oder 30**) aufgegeben.

In der Absorptionsstufe eingesetzte inerte Absorptionsmittel sind im Allgemeinen hochsiedende unpolare Lösungsmittel, in denen das abzutrennende C₄-Kohlenwasserstoff-Gemisch eine deutlich höhere Löslichkeit als die übrigen abzutrennenden Gasbestandteile aufweist. Geeignete Absorptionsmittel sind vergleichsweise unpolare organische Lösungsmittel, beispielsweise aliphatische C₈- bis C₁₈-Alkane, oder aromatische Kohlenwasserstoffe wie die Mittelölfraktionen aus der Paraffindestillation, Toluol oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁-C₈-Alkanolen, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl^{®}. Häufig enthält dieses Lösungsmittelgemisch Dimethylphthalat in einer Menge von 0,1 bis 25 Gew.-%.

Bevorzugte Absorptionsmittel sind Lösungsmittel, die ein Lösungsvermögen für organische Peroxide von mindestens 1000 ppm (mg aktiver Sauerstoff / kg Lösungsmittel) aufweisen. In der bevorzugten Ausführungsform wird als Lösungsmittel für die Absorption Toluol eingesetzt.

Falls eine kritischer Konzentration von Peroxide erreicht ist, welche zum Beispiel einer Zersetzungenergie der Lösung von größer 400 J/g, bevorzugt aber einer Zersetzungsenergie von 100 J/g entspricht, können Maßnahmen zur Reduzierung der Peroxidmenge in dem umlaufenden Absorptionsmittel durchgeführt werden. Diese Maßnahmen können ausgewählt sein aus einer Peroxid-Zerstörungen durch Anwendung erhöhter Temperaturen, wie von Hendry 1968 (luEC Product Research and Development, Vol.7,No.2, 1968, 136-145) beschrieben, Behandlung mit starken Basen, wie ebenfalls von Hendry 1968 (luEC Product Research and Development, Vol.7,No.2, 1968, 145-151) beschrieben, oder auch Hydrierreaktionen an heterogenen Katalysatoren. Hierzu können das die Peroxide enthaltende Absorptionsmittel erhitzt oder das Absorptionsmittel, beispielsweise in einem Rührbehälter, mit einer basischen wässrigen Lösung innig vermischt und die Phasen anschließend wieder getrennt werden, oder die in dem Absorptionsmittel enthaltenen Peroxide werden an einem heterogenen Festbettkatalysator hydriert.

Am Kopf der Absorptionskolonne **17** wird ein Abgasstrom **19** abgezogen, der im Wesentlichen Sauerstoff, leicht siedende Kohlenwasserstoffe (Methan, Ethan, Ethen, Propan, Propen), gegebenenfalls C₄-Kohlenwasserstoffe (Butan, Butene, Butadien), gegebenenfalls Inertgase, gegebenenfalls Kohlenstoffoxide und gegebenenfalls noch Wasserdampf enthält. Dieser Stoffstrom kann teilweise dem ODH-Reaktor zugeführt werden. Damit lässt sich zum Beispiel der Eintrittsstrom des ODH-Reaktors auf den gewünschten C₄-Kohlenwasserstoffgehalt einstellen.

Am Sumpf der Absorptionskolonne werden in einer weiteren Kolonne durch die Spülung mit einem Gas **18** Reste von im Absorptionsmittel gelöstem Sauerstoff ausgetragen. Der verbleibende Sauerstoffanteil ist vorzugsweise so gering, dass der die Desorptionskolonne verlassende, Butan, Buten sowie Butadien enthaltende Strom **31** nur noch maximal 100 ppm Sauerstoff enthält.

Das Ausstrippen des Sauerstoffs kann in jeder beliebigen, dem Fachmann bekannten geeigneten Kolonne durchgeführt werden. Das Strippen kann durch einfaches Durchleiten von nicht kondensierbaren Gasen durch die beladene Absorptionslösung erfolgen. Mit ausgestripptes C4 wird im oberen Teil der Absorptionskolonne **17** zurück in die Absorptionslösung gewaschen, indem der Gasstrom in diese Absorptionskolonne zurück geleitet wird. Das kann sowohl durch eine Verrohrung der Stripperkolonne als auch eine direkte Montage der Stripperkolonne unterhalb der Absorberkolonne erfolgen. Da der Druck im Strippkolonnenteil und Absorptionskolonnenteil erfindungsgemäß gleich ist, kann diese direkte Kopplung erfolgen. Geeignete Stippkolonnen sind z. B. Bodenkolonnen mit Glocken-, Zentrifugal- und/oder Siebboden, Kolonnen mit strukturierten Packungen, z. B. Blechpackungen mit einer spezifischen Oberfläche von 100 bis 1000 m²/m³ wie Mellapak® 250 Y, und Füllkörperkolonnen. Es kommen aber auch Riesel- und Sprühtürme sowie Rotationskolonnen, Tellerwäscher, Kreuzschleierwäscher und Rotationswäscher in Betracht. Geeignete Gase sind zum Beispiel Stickstoff oder Methan.

Der mit C₄-Kohlenwasserstoffen beladene Absorptionsmittelstrom **20** beinhaltet Wasser. Dieses wird in einem Dekanter **21** als Strom **22** vom Absorptionsmittel abgetrennt, so dass ein Strom **23** erhalten wird, der nur noch das im Absorptionsmittel eingelöste Wasser enthält.

Der mit C₄-Kohlenwasserstoffen beladene, weitestgehend vom Wasser befreite Absorptionsmittelstrom **23** kann in einem Wärmetauscher erwärmt werden und als Strom **24** anschließend in eine Desorptionskolonne **25** geleitet werden. In einer Verfahrensvariante wird der Desorptionsschritt Dc) durch Entspannung und/oder Erhitzen des beladenen Absorptionsmittels durchgeführt. Bevorzugte Verfahrensvariante ist die Nutzung eines Reboilers im Sumpf der Desorptionskolonne **25.**

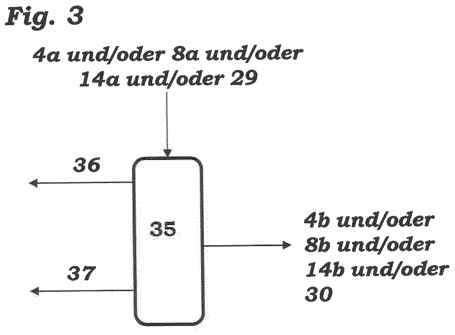

Das in der Desorptionsstufe regenerierte Absorptionsmittel **27** kann in einem Wärmetauscher abgekühlt werden und als Strom **28** in die Absorptionsstufe **17** zurückgeführt werden. Im Prozessgasstrom befindliche Leichtsieder wie beispielsweise Ethan oder Propan sowie schwersiedende Komponenten wie Benzaldehyd, Maleinsäure und Phthalsäure, können sich im Kreislaufstrom anreichern. Um die Anreicherung zu begrenzen, kann ein Purgestrom **29** abgezogen werden und dieser entweder wie auch die Ströme **14a**, **8b** und **4b** in einer Destillationskolonne **35** (Figur 3) nach dem Stand der Technik in Leichtsieder **36,** regeneriertes Absorbens **30** (Figur 2 und 3) und Schwersieder **37** aufgetrennt werden, oder bevorzugt den Strömen **14b, 8b** oder **4b** zugeführt werden, um die noch im Strom **29** gelösten C₄-Kohlenwasserstoffe in den Prozessgasstrom zurückzuwaschen. Wenn Strom **29** in der Destillationskolonne **35** getrennt wird, können die Ströme **36** und **37**verbrannt und somit energetisch genutzt werden.

In einer Ausführungsform der Erfindung wird der Peroxid-Gehalt in einem oder mehreren der Absorptionsmittelströmen **20, 23, 24, 27** bzw. **28** bestimmt, bevorzugt an einem der Absorptionsmittelströme **27** oder **28.**

Der im Wesentlichen aus n-Butan, n-Butenen und Butadien bestehende C₄-Produktgasstrom **31** enthält im Allgemeinen 20 bis 80 Vol.-% Butadien, 0 bis 80 Vol.-% n-Butan, 0 bis 10 Vol.-% 1-Buten, und 0 bis 50 Vol.-% 2-Butene, wobei die Gesamtmenge 100 Vol.-% ergibt. Weiterhin können geringe Mengen an Iso-Butan enthalten sein.

Ein Teil des kondensierten, hauptsächlich C₄-Kohlenwasserstoffe enthaltenen Kopfaustrags der Desorptionskolonne wird als Strom **34** in den Kolonnenkopf zurückgeführt, um die Trennleistung der Kolonne zu erhöhen.

Die C₄-Produktströme **32** und **33** werden anschließend durch Extraktivdestillation im Schritt E) mit einem für Butadien selektiven Lösungsmittel in einen Butadien und das selektive Lösungsmittel enthaltenden Stoffstrom und einen n-Butene enthaltenden Stoffstrom aufgetrennt.

Die Extraktivdestillation kann beispielsweise, wie in "Erdöl und Kohle - Erdgas - Petrochemie", Band 34 (8), Seiten 343 bis 346 oder "Ullmanns Enzyklopädie der Technischen Chemie", Band 9, 4. Auflage 1975, Seiten 1 bis 18 beschrieben, durchgeführt werden. Hierzu wird der C₄-Produktgasstrom mit einem Extraktionsmittel, vorzugsweise einem N-Methylpyrrolidon (NMP)/Wasser-Gemisch, in einer Extraktionszone in Kontakt gebracht. Die Extraktionszone ist im Allgemeinen in Form einer Waschkolonne ausgeführt, welche Böden, Füllkörper oder Packungen als Einbauten enthält. Diese weist im Allgemeinen 30 bis 70 theoretische Trennstufen auf, damit eine hinreichend gute Trennwirkung erzielt wird. Vorzugsweise weist die Waschkolonne im Kolonnenkopf eine Rückwaschzone auf. Diese Rückwaschzone dient zur Rückgewinnung des in der Gasphase enthaltenen Extraktionsmittels mit Hilfe eines flüssigen Kohlenwasserstoffrücklaufs, wozu die Kopffraktion zuvor kondensiert wird. Das Massenverhältnis Extraktionsmittel zu C₄-Produktgasstrom im Zulauf der Extraktionszone beträgt im Allgemeinen 10 : 1 bis 20 : 1. Die Extraktivdestillation wird vorzugsweise bei einer Sumpftemperatur im Bereich von 100 bis 250 °C, insbesondere bei einer Temperatur im Bereich von 110 bis 210 °C, einer Kopftemperatur im Bereich von 10 bis 100·°C, insbesondere im Bereich von 20 bis 70·°C und einem Druck im Bereich von 1 bis 15 bar, insbesondere im Bereich von 3 bis 8 bar betrieben. Die Extraktivdestillationskolonne weist vorzugsweise 5 bis 70 theoretische Trennstufen auf.

Geeignete Extraktionsmittel sind Butyrolacton, Nitrile wie Acetonitril, Propionitril, Methoxypropionitril, Ketone wie Aceton, Furfural, N-alkylsubstituierte niedere aliphatische Säureamide wie Dimethylformamid, Diethylformamid, Dimethylacetamid, Diethylacetamid, N-Formylmorpholin, N-alkylsubstituierte zyklische Säureamide (Lactame) wie N-Alkylpyrrolidone, insbesondere N-Methylpyrrolidon (NMP). Im Allgemeinen werden alkylsubstituierte niedere aliphatische Säureamide oder N-alkylsubstituierte zyklische Säureamide verwendet. Besonders vorteilhaft sind Dimethylformamid, Acetonitril, Furfural und insbesondere NMP.

Es können jedoch auch Mischungen dieser Extraktionsmittel untereinander, z.B. von NMP und Acetonitril, Mischungen dieser Extraktionsmittel mit Co-Lösungsmitteln und/oder tert.-Butylether, z.B. Methyl-tert.-butylether, Ethyl-tert.-butylether, Propyl-tert.-butylether, n- oder iso-Butyl-tert.-butylether eingesetzt werden. Besonders geeignet ist NMP, bevorzugt in wässriger Lösung, vorzugsweise mit 0 bis 20 Gew.-% Wasser, besonders bevorzugt mit 7 bis 10 Gew.-% Wasser, insbesondere mit 8,3 Gew.-% Wasser.

Der Kopfproduktstrom der Extraktivdestillationskolonne enthält im Wesentlichen Butan und Butene und in geringen Mengen Butadien und wird gasförmig oder flüssig abgezogen. Im Allgemeinen enthält der im Wesentlichen aus n-Butan und 2-Buten bestehende Strom bis 100 Vol-% n-Butan, 0 bis 50 Vol-% 2-Buten und 0 bis 3 Vol-% weitere Bestandteile wie Isobutan, Isobuten, Propan, Propen und C₅⁺-Kohlenwasserstoffe.

Der im Wesentlichen aus n-Butan und 2-Buten bestehende Strom kann ganz oder teilweise dem C₄-Feed des ODH-Reaktors zugeführt werden. Da die Buten-Isomere dieses Rückführstroms im Wesentlichen aus 2-Butenen bestehen, und 2-Butene im Allgemeinen langsamer zu Butadien oxidativ dehydriert werden als 1-Buten, kann dieser Rückführstrom vor der Zuführung in den ODH-Reaktor katalytisch isomerisiert werden. Dadurch kann die Isomerenverteilung entsprechend der im thermodynamischen Gleichgewicht vorliegenden Isomerenverteilung eingestellt werden.

In einem Schritt F) wird der Butadien und das selektive Lösungsmittel enthaltende Stoffstrom in einen im Wesentlichen aus dem selektiven Lösungsmittel bestehenden Stoffstrom und einen Butadien enthaltenden Stoffstrom destillativ aufgetrennt.

Der am Sumpf der Extraktivdestillationskolonne gewonnene Stoffstrom enthält im Allgemeinen das Extraktionsmittel, Wasser, Butadien und in geringen Anteilen Butene und Butan und wird einer Destillationskolonne zugeführt. In dieser kann über Kopf Butadien gewonnen werden. Am Sumpf der Destillationskolonne fällt ein Extraktionsmittel und gegebenenfalls Wasser enthaltender Stoffstrom an, wobei die Zusammensetzung des Extraktionsmittel und Wasser enthaltenden Stoffstroms der Zusammensetzung entspricht, wie sie der Extraktion zugegeben wird. Der Extraktionsmittel und Wasser enthaltende Stoffstrom wird bevorzugt in die Extraktivdestillation zurückgeleitet.

In einer Variante wird über einen Seitenabzug ein Butadien enthaltendes Extraktionsmittel abgezogen und in eine Desorptionszone überführt, wobei aus der Extraktionsmittellösung das Butadien desorbiert wird. Die Desorptionszone kann beispielsweise in Form einer Waschkolonne ausgeführt sein, die 2 bis 30, bevorzugt 5 bis 20 theoretische Stufen und gegebenenfalls eine Rückwaschzone mit beispielsweise 4 theoretischen Stufen aufweist. Diese Rückwaschzone dient zur Rückgewinnung des in der Gasphase enthaltenen Extraktionsmittels mit Hilfe eines flüssigen Kohlenwasserstoffrücklaufs aus Butadien, wozu die Kopffraktion kondensiert wird. Als Einbauten sind Packungen, Böden oder Füllkörper vorgesehen. Die Destillation wird vorzugsweise bei einer Sumpftemperatur im Bereich von 100 bis 300 °C, insbesondere im Bereich von 150 bis 200 °C und einer Kopftemperatur im Bereich von 0 bis 70 °C, insbesondere im Bereich von 10 bis 50 °C durchgeführt. Der Druck in der Destillationskolonne liegt dabei vorzugsweise im Bereich von 1 bis 10 bar. Im Allgemeinen herrschen in der Desorptionszone gegenüber der Extraktionszone ein verminderter Druck und/oder eine erhöhte Temperatur.

Der am Kolonnenkopf gewonnene Wertproduktstrom enthält im Allgemeinen 90 bis 100 Vol.-% Butadien, 0 bis 10 Vol.-% 2-Buten und 0 bis 10 Vol.-% n-Butan und iso-Butan. Zur weiteren Aufreinigung des Butadiens kann eine weitere Destillation nach dem Stand der Technik durchgeführt werden.

Die Erfindung wird durch das nachstehende Beispiel näher erläutert.

### Beispiel 1

Aus den Strömen 4, 8, 14 und 27 werden Proben entnommen und der kalorimetrischen Analyse mittels DSC zugeführt. Die DSC-Analyse läuft dabei wie folgt ab: Es werden 10 mg der Probemenge unter Inertgasatmosphäre in einen druckdichten Schraubtiegel aus V4A gegeben. Der Tiegel wird unter Inertgasatmosphäre gasdicht verschlossen und in das DSC-Kalorimeter gegeben. Ein zweiter, nicht gefüllter Tiegel wird als Referenztiegel eingesetzt. Beide Tiegel werden mit einer Temperaturrampe von 2,5 K/min aufgeheizt. Um beide Tiegel bei gleicher Temperatur zu halten, werden unterschiedliche Wärmeströme benötigt. Grund hierfür ist einerseits die unterschiedliche Wärmekapazität durch den unterschiedlichen Füllgrad der beiden Tiegel, andererseits die in dem gefüllten Tiegel stattfindenden endothermen und exothermen Reaktionen. Durch Integration der durch die Reaktion hervorgerufenen Differenzen in den Wärmeströmen über die Zeit kann die Reaktionswärme berechnet werden. Aus dieser wird unter Zugrundelegung der bekannten Zersetzungsenergie der Peroxide von 1340 J/g die Konzentration der Peroxide ermittelt.

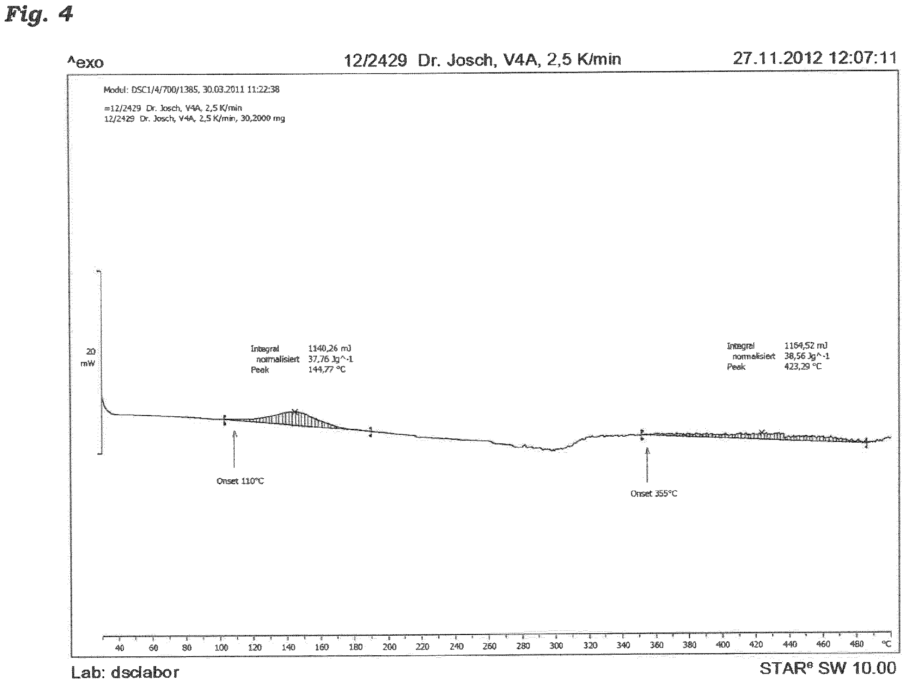

Figur 4 zeigt das DSC eines Toluolstroms, der Peroxie enthält. Aus der exothermen Reaktionswärme im für die Peroxidzersetzung charakteristischen Temperaturbereich um 140°C, die durch Integration zu 37,76 J/g bestimmt wurde, wurde eine Peroxid-Konzentration von 28 mg Peroxid / g Toluol berechnet. Die Zersetzungsenergie befindet sich noch nicht im deflagrativen Bereich, und damit stellen die in der Lösung enthaltenen Peroxide noch keine Gefahr dar.

### Beispiel 2:

Beim Betrieb des mit dem ODH-Reaktor gekoppelten Quenches wurden aus den Strömen 4 und 8 Proben gezogen und der Analyse zugeführt. Die Analyse umfasste sowohl die iodometrische Bestimmung von Peroxiden als auch die DSC-Analyse. Der Quench wurde in beiden Kolonnen 3 und 7 mit Mesitylen als Quench-Lösungsmittel betrieben.

Die DSC-Analyse lief hier wie folgt ab. Es wurden 3 bis 13 mg der Probemenge unter Inertgasatmosphäre in einen druckdichten Schraubtiegel gegeben. Der Tiegel wird unter Inertgasatmosphäre gasdicht verschlossen und in das DSC-Kalorimeter gegeben. Ein zweiter, aber ungefüllter Tiegel wird als Referenztiegel eingesetzt und beide mit einer Temperaturrampe von 2,5 K/min aufgeheizt. Um beide Tiegel, bei gleicher Temperatur zu halten, werden unterschiedliche Wärmeströme benötigt. Grund hierfür ist zum Einen, die unterschiedliche Wärmekapazität durch den unterschiedlichen Füllgrad der Tiegel und zum Anderen endotherme und exotherme Reaktionen der in den ersten Tiegel gefüllten Probenmenge. Durch Integration der durch die Reaktion hervorgerufenen Differenzen in den Wärmeströmen über die Zeit, kann die Reaktionswärme berechnet und aus dieser unter Zuhilfenahme der Zersetzungsenergie der Peroxide von 1340 J/g die Konzentration der Peroxide ermittelt werden.

Die Ergebnisse der iodometrischen Analyse zeigten für die Probe aus Strom 4 einen Wert von 1700 ppm Aktivsauerstoff (mg Aktivsauerstoff / kg Lösungsmittel) und für Strom 8 einen Wert von 407 ppm Aktivsauerstoff.

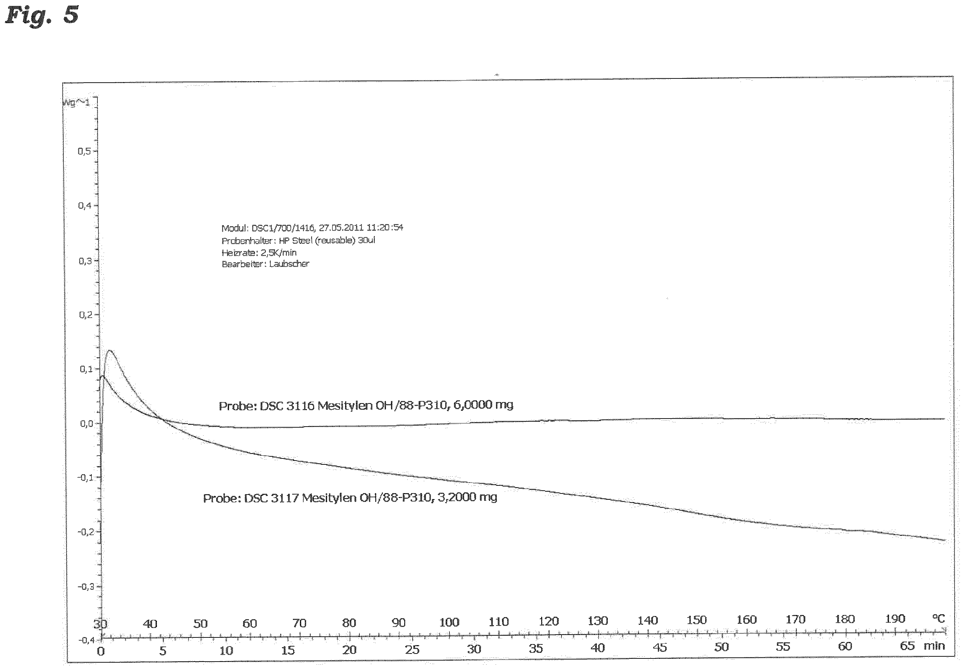

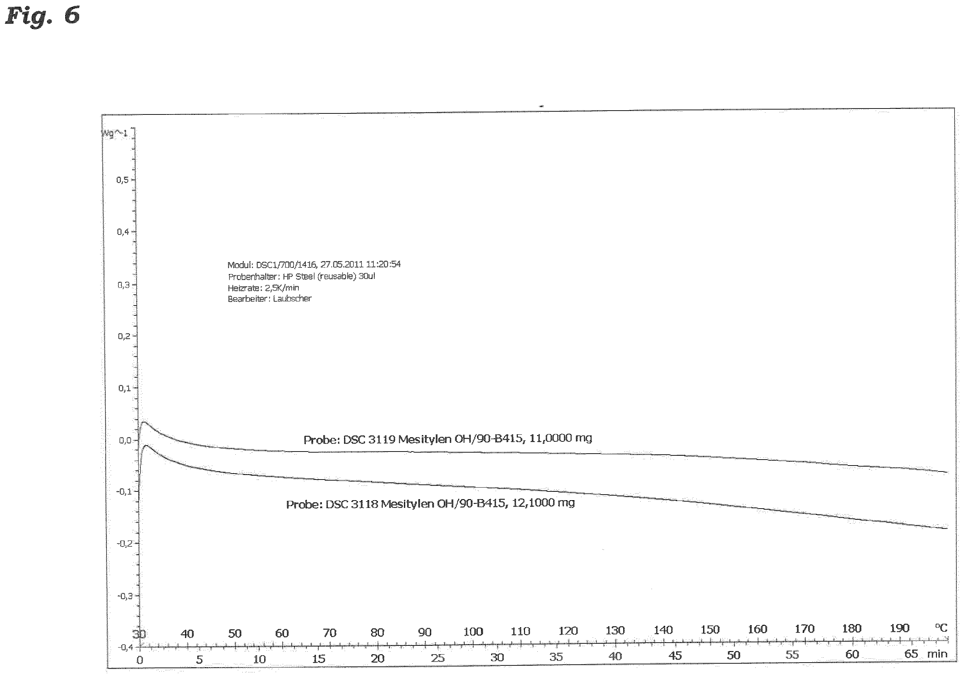

Demgegenüber wurden mittels DSC-Analyse bei beiden analysierten Strömen 4 und 8 keine Zersetzungsenergien und damit keine nennenswerten Mengen an selbstzersetzenden Substanzen wie Peroxide detektiert. Figur 5 zeigt das DSC-Ergebnis für Strom 4, während Figur 6 das Ergebnis für Strom 8 zeigt.

Die lodometrie detektiert folglich nicht nur Peroxide, sondern auch andere Komponenten, von denen jedoch kein Gefährdungspotential in Form von Selbstzersetzung ausgeht. Es wurde also nachgewiesen, dass die DSC die erfindungsgemäß als wesentlich zuverlässigere Analytik eingesetzt werden kann.

## Patentansprüche

1. Verfahren zur Herstellung von Butadien aus n-Butenen mit den folgenden Schritten:
A) Bereitstellung eines n-Butene enthaltenden Einsatzgasstroms a;
B) Einspeisung des n-Butene enthaltenden Einsatzgasstromes a und eines sauerstoffhaltigen Gases in mindestens eine Dehydrierzone und oxidative Dehydrierung von n-Butenen zu Butadien, wobei ein Produktgasstrom b enthaltend Butadien, nicht umgesetzte n-Butene, Wasserdampf, Sauerstoff, leicht siedende Kohlenwasserstoffe, gegebenenfalls Kohlenstoffoxide und gegebenenfalls Inertgase erhalten wird;
C) Abkühlung und Kompression des Produktgasstroms b in mindestens einer Abkühlstufe und mindestens einer Kompressionsstufe, wobei der Produktgasstrom b mit mindestens einem im Kreis geführtem organischen Lösungsmittel als Kühlmittel in Kontakt gebracht wird, wobei mindestens ein Kondensatstrom c1 enthaltend Wasser und ein Gasstrom c2 enthaltend Butadien, n-Butene, Wasserdampf, Sauerstoff, leicht siedende Kohlenwasserstoffe, gegebenenfalls Kohlenstoffoxide und gegebenenfalls Inertgase erhalten wird;
D) Abtrennung von nicht kondensierbaren und leicht siedenden Gasbestandteilen umfassend Sauerstoff, leicht siedende Kohlenwasserstoffe, gegebenenfalls Kohlenstoffoxide und gegebenenfalls Inertgase als Gasstrom d2 aus dem Gasstrom c2 durch Absorption der C₄-Kohlenwasserstoffe umfassend Butadien und n-Butene in mindestens einem im Kreis geführten Absorptionsmittel, wobei mindestens ein mit C₄-Kohlenwasserstoffen beladener Absorptionsmittelstrom und der Gasstrom d2 erhalten werden, und anschließende Desorption der C₄-Kohlenwasserstoffe aus dem beladenen Absorptionsmittelstrom, wobei ein C₄-Produktgasstrom d1 erhalten wird;
E) Auftrennung des C₄-Produktstroms d1 durch Extraktivdestillation mit einem für Butadien selektiven Lösungsmittel in einen Butadien und das selektive Lösungsmittel enthaltenden Stoffstrom e1 und einen n-Butene enthaltenden Stoffstrom e2;
F) Destillation des Butadien und das selektive Lösungsmittel enthaltenden Stoffstroms e1 in einen im Wesentlichen aus dem selektiven Lösungsmittel bestehenden Stoffstrom f1 und einen Butadien enthaltenden Stoffstrom f2;
wobei dem in Schritt C) im Kreis geführten Kühlmittel und optional dem in Schritt D) im Kreis geführten Absorptionsmittel Proben entnommen werden und in den entnommenen Proben mittels lodometrie, Differential Scanning Calometrie (DSC) oder Mikrokalorimetrie der Peroxid-Gehalt bestimmt wird, wobei bei einer Zersetzungsenergie des im Kreis geführten Kühlmittels von größer 400 J/g Peroxide in dem Kühlmittel durch Erhitzen des Kühlmittels, Behandlung des Kühlmittels mit Basen, oder Hydrierung vernichtet werden.

2. Verfahren nach Anspruch 1, **dadurch gekennzeichnet, dass** die Stufe C) mindestens eine Abkühlungsstufe Ca) und mindestens eine Kompressionsstufe Cb) umfasst.

3. Verfahren nach Anspruch 2, **dadurch gekennzeichnet, dass** die Abkühlstufe Ca) zweistufig in zwei Abkühlstufen Ca1) und Ca2) durchgeführt wird.

4. Verfahren nach Anspruch 3, **dadurch gekennzeichnet, dass** zumindest ein Teil des Kühlmittels nach Durchlaufen der zweiten Abkühlstufe Ca2) als Kühlmittel der ersten Abkühlstufe Ca1) zugeführt wird.

5. Verfahren nach einem der Ansprüche 2 bis 4, **dadurch gekennzeichnet, dass** die Stufe Cb) mindestens eine Kompressionsstufe Cba) und mindestens eine Abkühlstufe Cbb) umfasst, wobei in der mindestens einen Abkühlstufe Cbb) das in der Kompressionsstufe Cba) komprimierte Gas mit einem Kühlmittels in Kontakt gebracht wird.

6. Verfahren nach Anspruch 5, **dadurch gekennzeichnet, dass** das Kühlmittel der Abkühlstufe Cbb) das gleiche organische Lösungsmittel, das in der Abkühlstufe Ca) als Kühlmittel verwendet wird, ist und zumindest ein Teil dieses Kühlmittels nach Durchlaufen der mindestens einen Abkühlstufe Cbb) als Kühlmittel der Abkühlstufe Ca) zugeführt wird.

7. Verfahren nach einem der Ansprüche 1 bis 6, **dadurch gekennzeichnet, dass** das im Kreis geführte Kühlmittel Toluol ist.

8. Verfahren nach einem der Ansprüche 1 bis 7, **dadurch gekennzeichnet, dass** Schritt D) die Schritte Da) bis Dc) umfasst:
Da) Absorption der C₄-Kohlenwasserstoffe umfassend Butadien und n-Butene in einem Absorptionsmittel, wobei ein mit C₄-Kohlenwasserstoffen beladener Absorptionsmittelstrom und der Gasstrom d2 erhalten werden,
Db) Entfernung von Sauerstoff aus dem mit C₄-Kohlenwasserstoffen beladenen Absorptionsmittelstrom aus Schritt Da) durch Strippung mit einem nicht kondensierbaren Gasstrom, und
Dc) Desorption der C₄-Kohlenwasserstoffe aus dem beladenen Absorptionsmittelstrom, wobei ein C₄-Produktgasstrom d1 erhalten wird.

9. Verfahren nach einem der Ansprüche 1 bis 8, **dadurch gekennzeichnet, dass** das in Schritt D) verwendete Absorptionsmittel das gleiche organische Lösungsmittel wie ein in Schritt C) verwendete Kühlmittel ist, und zumindest ein Teil dieses Absorptionsmittels nach Desorption der C₄-Kohlenwasserstoffe als Kühlmittel dem Schritt C) zugeführt wird.

10. Verfahren nach einem der Ansprüche 1 bis 9, **dadurch gekennzeichnet, dass** das Absorptionsmittel Toluol ist.

11. Verfahren nach einem der Ansprüche 1 bis 10, **dadurch gekennzeichnet, dass** bei einer Zersetzungsenergie des im Kreis geführten Absorptionsmittels von größer 400 J/g in dem Absorptionsmittel Peroxide durch Erhitzen des Absorptionsmittels, Behandlung des Absorptionsmittels mit einer Base, oder Hydrierung vernichtet werden.

## Claims

1. A process for preparing butadiene from n-butenes, which comprises the following steps:
A) provision of a feed gas stream a comprising n-butenes;
B) introduction of the feed gas stream a comprising n-butenes and an oxygen-comprising gas into at least one dehydrogenation zone and oxidative dehydrogenation of n-butenes to butadiene, giving a product gas stream b comprising butadiene, unreacted n-butenes, water vapor, oxygen, low-boiling hydrocarbons, possibly carbon oxides and possibly inert gases;
C) cooling and compression of the product gas stream b in at least one cooling stage and at least one compression stage, with the product gas stream b being brought into contact with at least one circulated organic solvent as coolant to give at least one condensate stream c1 comprising water and a gas stream c2 comprising butadiene, n-butenes, water vapor, oxygen, low-boiling hydrocarbons, possibly carbon oxides and possibly inert gases;
D) separation of incondensable and low-boiling gas constituents comprising oxygen, low-boiling hydrocarbons, possibly carbon oxides and possibly inert gases as gas stream d2 from the gas stream c2 by absorption of the C₄-hydrocarbons comprising butadiene and n-butenes in at least one circulated absorption medium, giving at least one absorption medium stream loaded with C₄-hydrocarbons and the gas stream d2, and subsequent desorption of the C₄-hydrocarbons from the loaded absorption medium stream to give a C₄ product gas stream d1;
E) separation of the C₄ product stream d1 by extractive distillation using a solvent which is selective for butadiene into a stream e1 comprising butadiene and the selective solvent and a stream e2 comprising n-butenes;
F) distillation of the stream e1 comprising butadiene and the selective solvent to give a stream f1 consisting essentially of the selective solvent and a stream f2 comprising butadiene;
where samples are taken from the circulated coolant in step C) and optionally the circulated absorption medium in step D) and the peroxide content of the samples taken is determined by means of iodometry, differential scanning calorimetry (DSC) or microcalorimetry, where when the decomposition energy of the circulated coolant is greater than 400 J/g in the coolant, peroxides are destroyed by heating of the coolant, treatment of the coolant with bases or hydrogenation.

2. The process according to claim 1, wherein step C) comprises at least one cooling stage Ca) and at least one compression stage Cb).

3. The process according to claim 2, wherein the cooling stage Ca) is carried out in two cooling stages Ca1) and Ca2).

4. The process according to claim 3, wherein at least part of the coolant which has passed through the second cooling stage Ca2) is fed as coolant to the first cooling stage Ca1).

5. The process according to any of claims 2 to 4, wherein the stage Cb) comprises at least one compression stage Cba) and at least one cooling stage Cbb), where the gas which has been compressed in the compression stage Cba) is brought into contact with a coolant in the at least one cooling stage Cbb).

6. The process according to claim 5, wherein the coolant of the cooling stage Cbb) is the same organic solvent which is used as coolant in the cooling stage Ca) and at least part of this coolant is, after passing through the at least one cooling stage Cbb), fed as coolant to the cooling stage Ca) .

7. The process according to any of claims 1 to 6, wherein the circulated coolant is toluene.

8. The process according to any of claims 1 to 7, wherein step D) comprises the steps Da) to Dc):
Da) absorption of the C₄-hydrocarbons comprising butadiene and n-butenes in an absorption medium to give an absorption medium stream loaded with C₄-hydrocarbons and the gas stream d2,
Db) removal of oxygen from the absorption medium stream loaded with C₄-hydrocarbons from step Da) by stripping with an incondensable gas stream and
Dc) desorption of the C₄-hydrocarbons from the loaded absorption medium stream to give a C₄ product gas stream d1.

9. The process according to any of claims 1 to 8, wherein the absorption medium used in step D) is the same organic solvent as a coolant used in step C) and at least a part of this absorption medium is, after desorption of the C₄-hydrocarbons, fed as coolant to step C).

10. The process according to any of claims 1 to 9, wherein the absorption medium is toluene.

11. The process according to any of claims 1 to 10, wherein when a decomposition energy of the circulated absorption medium is greater than 400 J/g in the absorption medium, peroxides are destroyed by heating of the absorption medium, treatment of the absorption medium with a base or hydrogenation.

## Revendications

1. Procédé pour la préparation de butadiène à partir de n-butènes présentant les étapes suivantes :
A) mise à disposition d'un flux gazeux de départ a contenant des n-butènes ;
B) injection du flux gazeux de départ a contenant des n-butènes et d'un gaz contenant de l'oxygène dans au moins une zone de déshydrogénation et déshydrogénation oxydante de n-butènes en butadiène, un flux gazeux produit b, contenant du butadiène, des n-butènes non transformés, de la vapeur d'eau, de l'oxygène, des hydrocarbures à bas point d'ébullition, le cas échéant des oxydes de carbone et le cas échéant des gaz inertes, étant obtenu ;
C) refroidissement et compression du flux gazeux produit b dans au moins une étape de refroidissement et au moins une étape de compression, le flux gazeux produit b étant mis en contact avec au moins un solvant organique guidé en circulation comme agent de refroidissement, au moins un flux de condensat c1 contenant de l'eau et un flux gazeux c2 contenant du butadiène, des n-butènes, de la vapeur d'eau, de l'oxygène, des hydrocarbures à bas point d'ébullition, le cas échéant des oxydes de carbone et le cas échéant des gaz inertes, étant obtenus ;
D) séparation, du flux gazeux c2, des constituants gazeux non condensables et de bas point d'ébullition, comprenant de l'oxygène, des hydrocarbures à bas point d'ébullition, le cas échéant des oxydes de carbone et le cas échéant des gaz inertes, sous forme de flux gazeux d2, par absorption des hydrocarbures en C₄ comprenant du butadiène et des n-butènes dans au moins un absorbant guidé en circulation, au moins un flux d'absorbant chargé d'hydrocarbures en C₄ et le flux gazeux d2 étant obtenus et désorption consécutive des hydrocarbures en C₄ du flux d'absorbant chargé, un flux gazeux produit en C₄ d1 étant obtenu ;
E) séparation du flux gazeux produit en C₄ d1 par distillation extractive à l'aide d'un solvant sélectif pour le butadiène en un flux de substances e1 contenant du butadiène et le solvant sélectif et en un flux de substances e2 contenant des n-butènes ;
F) distillation du flux de substances e1, contenant du butadiène et le solvant sélectif, en un flux de substances f1, essentiellement constitué par le solvant sélectif, et en un flux de substances f2 contenant du butadiène ;
des échantillons étant prélevés de l'agent de refroidissement guidé en circulation dans l'étape C) et éventuellement de l'absorbant guidé en circulation dans l'étape D) et la teneur en peroxyde étant déterminée dans les échantillons prélevés par iodométrie, par calorimétrie différentielle à balayage (DSC) ou par microcalorimétrie, les peroxydes dans l'agent de refroidissement étant détruits, à une énergie de décomposition de l'agent de refroidissement supérieure à 400 J/g, par chauffage de l'agent de refroidissement, par traitement de l'agent de refroidissement par des bases ou par hydrogénation.

2. Procédé selon la revendication 1, **caractérisé en ce que** l'étape C) comprend au moins une étape de refroidissement Ca) et au moins une étape de compression Cb).

3. Procédé selon la revendication 2, **caractérisé en ce que** l'étape de refroidissement Ca) est réalisée en deux étapes dans deux étapes de refroidissement Ca1) et Ca2).

4. Procédé selon la revendication 3, **caractérisé en ce qu'**au moins une partie de l'agent de refroidissement, après avoir traversé la deuxième étape de refroidissement Ca2), est introduite comme agent de refroidissement dans la première étape de refroidissement Ca1).

5. Procédé selon l'une quelconque des revendications 2 à 4, **caractérisé en ce que** l'étape Cb) comprend au moins une étape de compression Cba) et au moins une étape de refroidissement Cbb), le gaz comprimé dans l'étape de compression Cba) étant mis en contact, dans ladite au moins une étape de refroidissement Cbb), avec un agent de refroidissement.

6. Procédé selon la revendication 5, **caractérisé en ce que** l'agent de refroidissement de l'étape de refroidissement Cbb) est le même solvant organique que celui qui est utilisé dans l'étape de refroidissement Ca) comme agent de refroidissement et au moins une partie de cet agent de refroidissement est introduite, après avoir traversé ladite au moins une étape de refroidissement Cbb), comme agent de refroidissement dans l'étape de refroidissement Ca).

7. Procédé selon l'une quelconque des revendications 1 à 6, **caractérisé en ce que** l'agent de refroidissement guidé en circulation est le toluène.

8. Procédé selon l'une quelconque des revendications 1 à 7, **caractérisé en ce que** l'étape D) comprend les étapes Da) à Dc) :
Da) absorption des hydrocarbures en C₄ comprenant du butadiène et des n-butènes dans un absorbant, un flux d'absorbant chargé d'hydrocarbures en C₄ et le flux gazeux d2 étant obtenus,
Db) élimination de l'oxygène du flux d'absorbant chargé en hydrocarbures en C₄ de l'étape Da) par extraction à l'aide d'un flux gazeux non condensable et
Dc) désorption des hydrocarbures en C₄ du flux d'absorbant chargé, un flux gazeux produit en C₄ d1 étant obtenu.

9. Procédé selon l'une quelconque des revendications 1 à 8, **caractérisé en ce que** l'absorbant utilisé dans l'étape D) est le même solvant organique qu'un agent de refroidissement utilisé dans l'étape C) et au moins une partie de cet absorbant est introduite après désorption des hydrocarbures en C₄ comme agent de refroidissement dans l'étape C).

10. Procédé selon l'une quelconque des revendications 1 à 9, **caractérisé en ce que** l'absorbant est le toluène.

11. Procédé selon l'une quelconque des revendications 1 à 10, **caractérisé en ce qu'**à une énergie de décomposition de l'absorbant guidé en circulation supérieure à 400 J/g, les peroxydes dans l'absorbant sont détruits par chauffage de l'absorbant, par traitement de l'absorbant par une base ou par hydrogénation.
